(19) Europäisches Patentamt
European Patent Office
Office européen des brevets

(11) Veröffentlichungsnummer: **0 379 085 B1**

(12) # EUROPÄISCHE PATENTSCHRIFT

(45) Veröffentlichungstag der Patentschrift: **05.07.95**

(51) Int. Cl.⁶: **C07D 211/44**, C07D 211/14, C07D 265/30, C07D 295/03, A01N 43/40, A01N 43/84

(21) Anmeldenummer: **90100615.5**

(22) Anmeldetag: **12.01.90**

Die Akte enthält technische Angaben, die nach dem Eingang der Anmeldung eingereicht wurden und die nicht in dieser Patentschrift enthalten sind.

(54) **Phenylalkylamine und diese enthaltende Fungizide.**

(30) Priorität: **18.01.89 DE 3901244**

(43) Veröffentlichungstag der Anmeldung:
**25.07.90 Patentblatt 90/30**

(45) Bekanntmachung des Hinweises auf die Patenterteilung:
**05.07.95 Patentblatt 95/27**

(84) Benannte Vertragsstaaten:
**AT BE CH DE DK ES FR GB GR IT LI NL SE**

(56) Entgegenhaltungen:
**EP-A- 0 164 706**
**EP-A- 0 244 739**
**EP-A- 0 262 870**

**CHEMICAL ABSTRACTS, Band 94, Nr. 21, 25. Mai 1981, Seite 735, Zusammenfassung Nr. 175136s, Columbus, Ohio, US;**

(73) Patentinhaber: **BASF Aktiengesellschaft**
**Carl-Bosch-Strasse 38**
**D-67063 Ludwigshafen (DE)**

(72) Erfinder: **Zipplies, Matthias, Dr.**
**Kastanienweg 1**
**D-6945 Hirschberg (DE)**
Erfinder: **Buschmann, Ernst, Dr.**
**Georg-Ludwig-Krebs-Strasse 10**
**D-6700 Ludwigshafen (DE)**
Erfinder: **Ammermann, Eberhard, Dr.**
**Sachsenstrasse 3**
**D-6700 Ludwigshafen (DE)**
Erfinder: **Lorenz, Gisela, Dr.**
**Erlenweg 13**
**D-6730 Neustadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 379 085 B1

## Beschreibung

Die vorliegende Erfindung betrifft Phenylalkylamine(-morpholine, -piperidine), diese enthaltende Fungizide und Verfahren zur Bekämpfung von Pilzen mit diesen Verbindungen.

Die EP-A 262 870 beschreibt bestimmte Morpholine und Piperidine, in denen der Heterocyclus über das Stickstoffatom mittels einer $(CH_2)_3$-Kette an eine Phenylgruppe gebunden ist. Hinsichtlich ihrer fungiziden Wirkung vermögen diese Verbindungen jedoch noch nicht zu befriedigen.

Aus der EP-A 164 706 ist die Verbindung II als Substanz mit das Pflanzenwachstum regulierenden Eigenschaften bekannt.

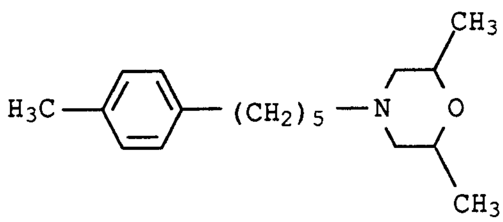

II

Die Verbindung wird nicht als fungizid wirksam beschrieben.

Es wurde nun gefunden, daß Phenylalkylamine der Formel I

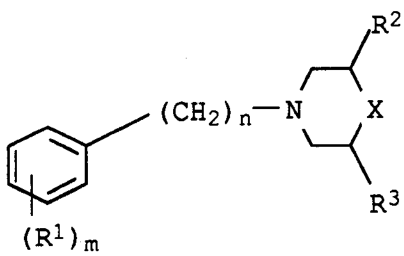

I

in der

- n — den Wert 5, 6, 7, 8 oder 9 hat,
- m — den Wert 1, 2 oder 3 hat,
- $R^1$ — Methyl, Halogen, Phenyl, welches bis zu drei $C_1$-$C_4$-Alkoxygruppen tragen kann, oder Phenoxy bedeutet, welches bis zu drei der folgenden Gruppen tragen kann: Halogen, $C_1$-$C_4$-Alkyl und $C_1$-$C_4$-Alkoxy,
  ferner, für den Fall, daß m = 2 ist, zwei benachbarte Reste $R^1$ zusammen den Rest

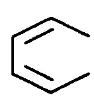

  bedeuten,
- X — Sauerstoff bedeutet, außer wenn $R^1$ Methyl oder Chlor bedeutet, oder
- X — den Rest $=CR^4R^5$ bedeutet, wobei
- $R^4$ — Isopropyl, tert.-Butyl oder Phenyl bedeutet, welches bis zu drei der folgenden Gruppe tragen kann: Halogen, $C_1$-$C_4$-Alkyl und $C_1$-$C_4$-Alkoxy, und
- $R^5$ — H oder OH bedeutet,
- $R^2$,$R^3$ — Wasserstoff bedeuten und
- $R^2$,$R^3$ — für den Fall, daß X Sauerstoff bedeutet, zusätzlich Methyl oder Ethyl bedeuten,

sowie deren pflanzenverträglichen Salze eine ausgezeichnete fungizide Wirkung gegen phytopathogene Pilze besitzen und dabei jedoch nicht phytotoxisch wirken.

$R^1$ bedeutet beispielsweise Chlor, Brom oder Jod oder beispielsweise gegebenenfalls bis zu dreifach durch Methoxy substituiertes Phenyl oder beispielsweise gegebenenfalls bis zu dreifach durch Methyl und/oder Methoxy substituiertes Phenoxy.

Für m = 2 bedeuten zwei benachbarte Reste $R^1$ zusammen den Rest

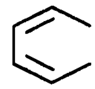

d.h. zusammen mit dem Phenylrest bedeuten sie den Naphthylrest

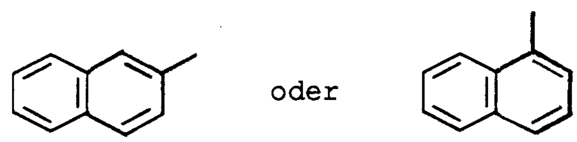

$R^4$ bedeutet beispielsweise einen gegebenenfalls bis zu dreifach substituierten Phenylrest mit den Substituenten $C_1$-$C_4$-Alkyl (Methyl), Halogen, $C_1$-$C_4$-Alkoxy (Methoxy).

Unter Salzen sind Salze mit beliebigen Protonen-aciden Verbindungen, anorganischen und organischen Säuren zu verstehen, z.B mit Chlorwasserstoff, Fluorwasserstoff, Bromwasserstoff, Schwefelsäure, Phosphorsäure, Jodwasserstoff, Dodecylbenzolsulfonsäure, Ameisensäure, Alkylcarbonsäure, Essigsäure, Propionsäure, Palmitinsäure, Perfluorheptansäure, Glycerin-2-phosphorsäure, Methylschwefelsäure, Methansulfonsäure, p-Toluolsulfonsäure, Salpetersäure, 2,6-Dichlorisonicotinsäure, Saccharin, ferner beispielsweise die folgenden Salze: Hydrogensulfat, Dihydrogenphosphat.

Die neuen Phenylalkylamine der Formel I enthalten teilweise chirale Zentren. Sie werden bei ihrer Herstellung im allgemeinen als Racemate oder gegebenenfalls als Diastereomerengemische erhalten.

Reine diastereomere Verbindungen lassen sich bei einigen der neuen Verbindungen beispielsweise durch Destillation, Säulenchromatographie oder aufgrund von Löslichkeitsunterschieden isolieren. Enantiomerenreine Verbindungen lassen sich z.B. durch Racematspaltung mit einem chiralen Hilfsreagenz nach bekannten Methoden, beispielsweise über diastereomere Salze, erhalten. Für die Anwendung der neuen Phenylalkylamine I als Fungizide sind sowohl die Diastereomeren bzw. die Enantiomeren als auch deren bei der Synthese anfallenden Stereoisomerengemische geeignet. Sie alle werden von der Erfindung umfaßt.

Bedeuten in dem Rest

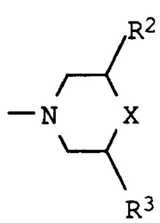

X = Sauerstoff und $R^2$, $R^3$ = Methyl und/oder Ethyl, so sind diejenigen Verbindungen besonders bevorzugt, in denen $R^2$ und $R^3$ cis zueinander stehen.

Die erfindungsgemäßen Verbindungen I lassen sich auf an sich bekannte Weise herstellen (Gleichung 1),

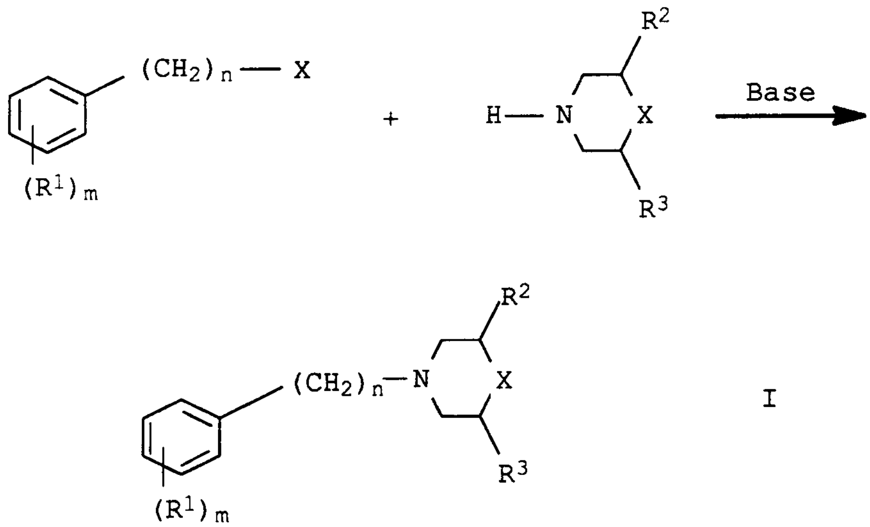


(1)

I

indem man eine geeignete Phenylalkylverbindung der Struktur III, worin X eine nukleophil substituierbare Austrittsgruppe wie beispielsweise Chlor, Brom, die Mesyl- oder Toluolsulfonylgruppe bedeutet, mit einem geeignet substituierten sekundären Amin der Struktur IV in Gegenwart einer Hilfsbase, ggf. in Anwesenheit eines Lösungsmittels, umsetzt und das Reaktionsprodukt auf übliche Weise isoliert.

Als Lösungsmittel geeignet sind beispielsweise Tetrahydrofuran, Acetonitril, Toluol, Xylol, DMF, Methanol, Dimethylsulfoxid. Die Reaktion wird vorteilhaft durchgeführt bei einer Temperatur zwischen 50 und 200 °C. Als Hilfsbasen geeignet sind beispielsweise $Na_2CO_3$, $K_2CO_3$, $NaHCO_3$, Triethylamin oder aber auch ein Überschuß des sekundären Amins IV.

Die als Edukte benötigten Phenylalkylhalogenide der allg. Struktur III lassen sich auf an sich bekannte Weise durch Lithiumcuprat-katalysierte
Verknüpfung von Aryl- oder Arylalkylgrignard-Verbindungen mit Dihalogenalkanen V herstellen (Gl. 2). HAL = Cl, Br.

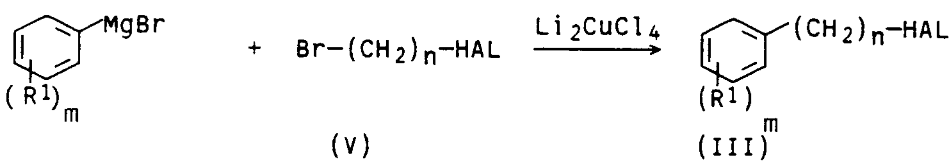


(V)

(III)

(2)

Phenylalkylhalogenide III mit Halogensubstitution im Aromaten ($R^1$ = Hal) lassen sich außer durch Kernhalogenierung von unsubstituierten Ausgangsverbindungen IIIa (Gl. 3)

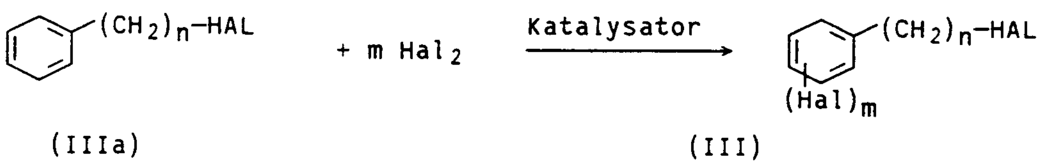


(IIIa)

(III)

(3)

auch über die in Gleichung 4 gezeigte allgemeine Reaktionssequenz herstellen (vgl. A. J. Hubert, J. Chem. Soc. C 1967, 235.).

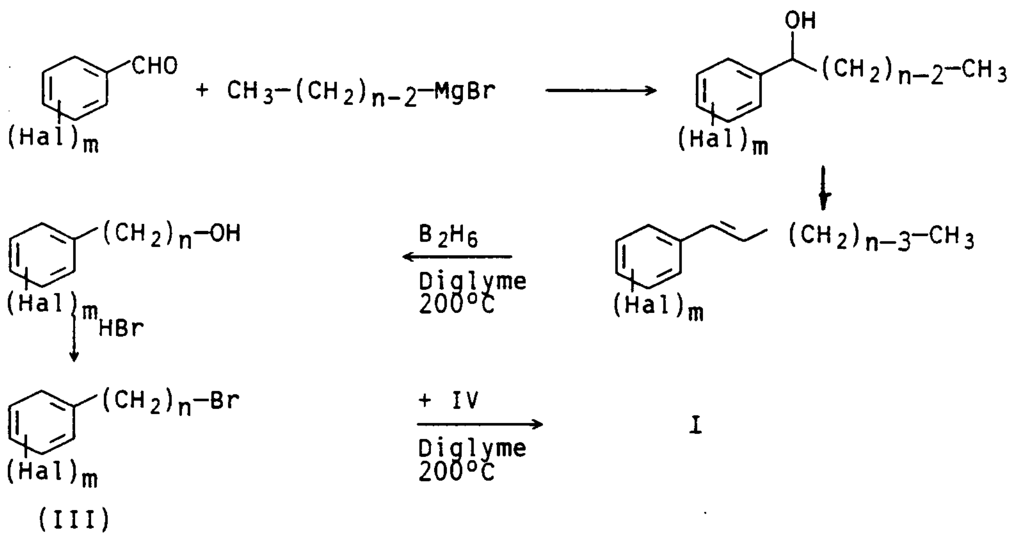


(4)

Kerniodierte Edukte I ($R^1$ = Jod) lassen sich beispielsweise nach dem allgemeinen Verfahren von Suzuki herstellen (Bull. Chem. Soc. Jap. 39, 128 (1966); Gleichung 5).

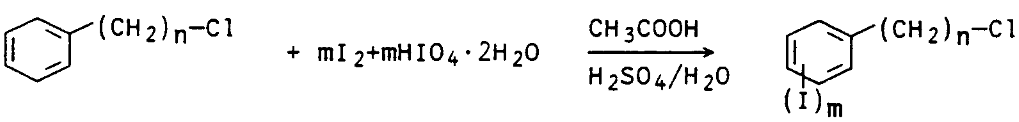


(5)

Herstellungsbeispiel für ein Vorprodukt

5-(4-Tolyl)-pentylbromid

Eine aus 148 g (0,87 mol) 4-Bromtoluol in 520 ml abs. Tetrahydrofuran und 22 g (0,92 mol) Magnesiumspänen bereitete Grignard -Lsg. wird bei -5 - 0 °C in 2 Std. zu einer unter Stickstoff gerührten Mischung aus 300 g (1,3 mol) 1,5-Dibrompentan, 1,5 g LiCl und 3,0 g $CuCl_2$ in 480 ml abs. Tetrahydrofuran getropft. Es wird 1 Std. bei Raumtemperatur nachgerührt, mit 1 100 ml ges. $NH_4Cl$-Lsg. versetzt, über Nacht bei Raumtemperatur (20 °C) gerührt und mit Ether extrahiert. Die Etherphase wird gewaschen, getrocknet, eingeengt und i. Vak. destilliert (140 - 45 °C/3,5 mbar): 79,6 g (38 % d. Th.).

Auf analoge Weise lassen sich eine Vielzahl anderer Edukte der allg. Struktur III herstellen.

Herstellungsbeispiele für neue Verbindungen

Beispiel 1

N-8-(4-Tolyl)-octyl-4-hydroxy-4-phenyl-piperidin (Verb. Nr. 1 der Tabelle)

10 g (40 mmol) 1-Chlor-8-tolyl-octan, 21,2 g (120 mmol) 4-Hydroxy-4-phenyl-piperidin, 5,52 g (40 mmol) Kaliumcarbonat und 3,3 g (20 mmol) Kaliumiodid werden mit 30 ml Xylol 8 Std. auf 160 °C erhitzt. Das Lösungsmittel wird i. Vak. abdestilliert. Der Rückstand wird mit Dichlormethan/verd. Natronlauge aufgenommen und der organische Extrakt wie üblich aufgearbeitet. Die leichtsiedenden Verbindungen werden i. Vak. bis 150 °C /0,9 mbar abdestilliert. Man erhält 11,4 g (94 %) eines Harzes (Physikalische Daten; siehe Tab. 1).

Auf die angegebene Art und Weise lassen sich die in Tabelle 1 aufgeführten neuen Verbindungen der Formel I herstellen.

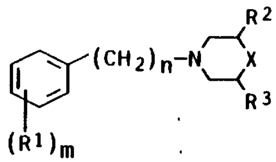 I

| Verb. Nr. | $(R^1)_m$ | n | X | $R^2$ | $R^3$ | Schmp.(°C)Sdp.(°C/mbar) IR($cm^{-1}$) | Bemerkungen |
|---|---|---|---|---|---|---|---|
| 1 | 4-$CH_3$ | 8 | 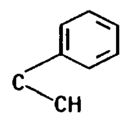 | H | H | 2924, 2849, 1975, 1378, 1110, 1042, 811, 758, 697, 547 | |
| 2 | 4-$CH_3$ | 5 | 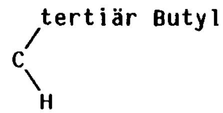 | H | H | 150/0,5 | |
| 3 | 4-$CH_3$ | 8 | 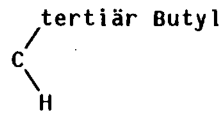 | H | H | 2921, 2850, 1515, 1463, 1450, 1364, 1124, 806, 491 | |
| 4 | 2,4-$Br_2$ | 5 | 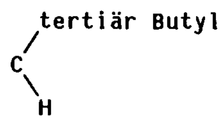 | H | H | 2940, 2861, 2802, 2767, 1467, 1393, 1377, 1364, 1126, 808 | |
| 5 | 4-Br | 5 | 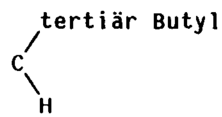 | H | H | 2932, 2855, 2800, 2764, 1487, 1467, 1443, 1122, 1353, 1040 | |
| 6 | 4-J | 5 | 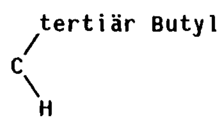 | H | H | 2962, 2942, 2934, 2855, 2646, 2504, 1485, 1474, 1467, 1007 | Hydrochlorid |

| Verb. Nr. | $(R^1)_m$ | n | X | $R^2$ | $R^3$ | Schmp.(°C)Sdp.(°C/mbar) IR($cm^{-1}$) | Bemerkungen |
|---|---|---|---|---|---|---|---|
| 7 | 2,4-$J_2$ | 5 | 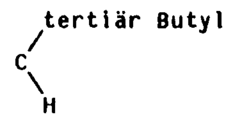 | H | H | 2956, 2854, 2715, 2640, 2571, 2498, 1456, 1437, 1394, 1363 | Hydrochlorid |
| 8 | 2,4,6-$(CH_3)_3$ | 5 | 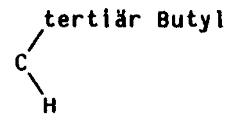 | H | H | 2942, 2860, 2801, 2767, 1480, 1468, 1376, 1364, 1127, 850 | |
| 9 | 4-Br | 5 | O | $CH_3$ | $CH_3$ | 162/0,15 | |
| 10 | 2,4-$Br_2$ | 5 | O | $CH_3$ | $CH_3$ | 162/0,15 | |
| 11 | 4-J | 5 | O | $CH_3$ | $CH_3$ | 160/0,2 | |
| 12 | 4-$C_6H_5$ | 5 | O | H | H | 2933, 2854, 2806, 1986, 1448, 1135, 1119, 868, 762, 698 | |
| 13 | 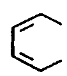 | 5 | O | $CH_3$ | $CH_3$ | 2970, 2933, 2857, 2811, 2773, 1323, 1145, 1086, 816, 746 | |
| 14 | 4-$CH_3$ | 5 | 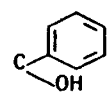 | H | H | 65 | |
| 15 | 2,4-$Br_2$ | 5 | 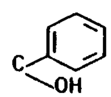 | H | H | 2939, 2858, 2822, 1464, 1446, 1377, 1118, 1045, 761, 700 | |
| 16 | 3,4-$(CH_3)_2$ | 5 | 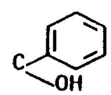 | H | H | 2927, 2850, 2823, 1469, 1445, 1381, 1113, 1009, 760, 698 | |

| Verb. Nr. | $(R^1)_m$ | n | X | $R^2$ | $R^3$ | Schmp. (°C) Sdp. (°C/mbar) IR($cm^{-1}$) | Bemerkungen |
|---|---|---|---|---|---|---|---|
| 17 | 2,4,6-$(CH_3)_3$ | 5 | 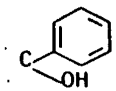 | H | H | 85-88 | |
| 18 | 4-$C_6H_5$ | 5 | 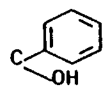 | H | H | 78-80 | |
| 19 | 2-$CH_3$ | 5 | 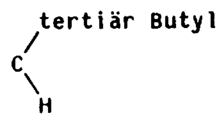 | H | H | 180/0,2 | |
| 20 | 2-$CH_3$ | 5 | 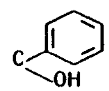 | H | H | Harz 2939, 2917, 2818, 1460, 1443, 1145, 1045, 760, 738, 703 | |
| 21 | 2,4-$Cl_2$ | 5 | 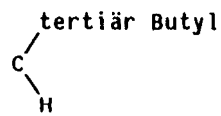 | H | H | 151/0,2 | |
| 22 | 2,4,6-$Br_3$ | 5 | O | $CH_3$ | $CH_3$ | 99-101 | |
| 23 | 2,4-$Cl_2$ | 5 | 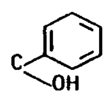 | H | H | 80-85 | |

| Verb. Nr. | $(R^1)_m$ | n | X | $R^2$ | $R^3$ | Schmp.(°C)Sdp.(°C/mbar) IR($cm^{-1}$) | Bemerkungen |
|---|---|---|---|---|---|---|---|
| 24 | $4-C_6H_5-O$ | 5 | O | $CH_3$ | $CH_3$ | 180/0,2 | |
| 25 | $4-C_6H_5-O$ | 5 | 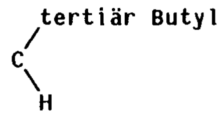 | H | H | 205/0,2 | |
| 26 | $4-C_6H_5-O$ | 5 | 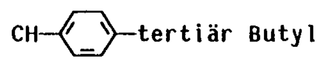 | H | H | 2933, 2858, 1590, 1506, 1489, 1469, 1240, 871, 821, 691 | |
| 27 | $4-C_6H_5-O$ | 5 | $CH-C_6H_5$ | H | H | 2932, 2855, 1590, 1506, 1489, 1239, 871, 755, 699, 691 | |
| 28 | $4-C_6H_5-O$ | 5 | 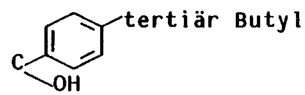 | H | H | 115 | |
| 29 | $4-C_6H_5-O$ | 5 | 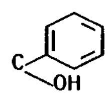 | H | H | | |
| 30 | $4-C_6H_5-O$ | 6 | 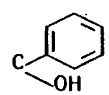 | H | H | | |
| 31 | $4-C_6H_5-O$ | 7 | 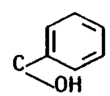 | H | H | | |
| 32 | $4-C_6H_5-O$ | 8 | 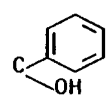 | H | H | | |

| Verb. Nr. | $(R^1)_m$ | n | X | $R^2$ | $R^3$ | Schmp.(°C)Sdp.(°C/mbar) IR($cm^{-1}$) | Bemerkungen |
|---|---|---|---|---|---|---|---|
| 33 | 2,4-$Cl_2$ | 6 | 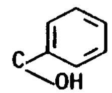 | H | H | | |
| 34 | 2,4-$Cl_2$ | 8 | 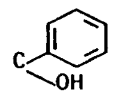 | H | H | | |
| 35 | 2,4-$Cl_2$ | 6 | 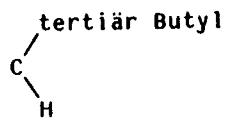 | H | H | | |
| 36 | 2,4-$Cl_2$ | 8 | 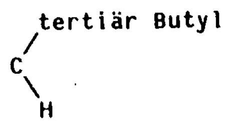 | H | H | | |
| 37 | 2,4-$Br_2$ | 6 | O | $CH_3$ | $CH_3$ | | |
| 38 | 2,4-$Br_2$ | 8 | O | $CH_3$ | $CH_3$ | | |
| 39 | 2,4-$Br_2$ | 6 | 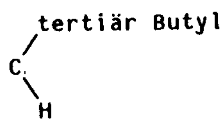 | H | H | | |
| 40 | 2,4-$Br_2$ | 8 | 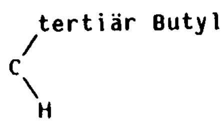 | H | H | | |

| Verb. Nr. | $(R^1)_m$ | n | X | $R^2$ | $R^3$ | Schmp.(°C) Sdp.(°C/mbar) IR($cm^{-1}$) | Bemerkungen |
|---|---|---|---|---|---|---|---|
| 41 | 2,4-$Br_2$ | 6 | 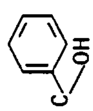 | H | H | | |
| 42 | 2,4-$Br_2$ | 8 | 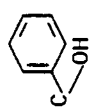 | H | H | | |
| 43 | 2,4-$Br_2$ | 5 | 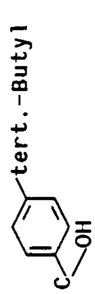 | H | H | | |
| 44 | 2,4-$Br_2$ | 5 | 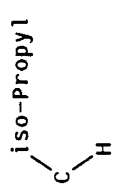 | H | H | | |
| 45 | 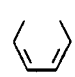 | 5 | 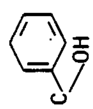 | H | H | | |
| 46 | 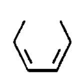 | 6 | 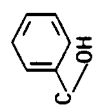 | H | H | | |

Die neuen Verbindungen I zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis,

Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 2 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Hr. 7 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 9 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 10 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr.13 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr.15 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr.17 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr.18 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr.21 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithiolo-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,

N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-lod-benzoesäure-anilid,
N-Formyl-N-morpholin-2,2,2-trichlorethylacetal,
Piperazin-1,4-diylbis(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoffe wurden N-5-(4-Methylphenyl)-n-pentyl-2,6-dimethylmorpholin (A) (Formel II, s. S.1) - bekannt aus EP 164 706 - und N-(3-(p-tertiär-Butylphenyl)-2-methylpropyl)-cis-2,6-dimethylmorpholin (B) - bekannt aus DE-2 656 747.5 - benutzt.

Anwendungsbeispiel 1

Wirksamkeit gegen Gurkenmehltau (kurativ)

Junge Gurkenpflanzen der Sorte "Chinesische Schlange" wurden im Zweiblattstadium mit einer wäßrigen Konidiensuspension des Gurkenmehltaus (Eryisphe cichoracearum und Sphaerotheca fuliginea) besprüht. Am nächsten Tag wurden diese Pflanzen mit wäßriger Spritzbrühe, die 80 % Wirkstoff und 20 % Emulgiermittel in der Trockensubstanz enthielt bis zur Tropfnässe besprüht und im Gewächshaus bei Temperaturen zwischen 20 und 22 °C und 70 - 80 % Luftfeuchtigkeit aufgestellt. 21 Tage nach der Wirkstoffapplikation wurde das Ausmaß der Pilzbefalls ermittelt.

Das Ergebnis zeigt, daß die Wirkstoffe 2, 7, 9, 10, 13, 15, 17, 18, 21, 22, 24 und 27 bei der Anwendung als 0,025 %ige (Gew.%) Spritzbrühe eine bessere fungizide Wirkung zeigen (90 %) als die bekannten Vergleichswirkstoffe A (55 %) und B (55 %).

Anwendungsbeispiel 2

Wirksamkeit gegen Rebenperonospora

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) inoculiert. Danach wurden die Reben zunächst für 48 Stunden in einer wasserdampfgesättigten Kammer bei 24 °C und anschließend für 5 Tage in einem Gewächshaus mit Temperaturen zwischen 20 und 30 °C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 1, 2, 3, 4, 5, 6, 7, 9, 11, 14, 15, 16, 18, 19, 20, 21, 23 und 26 bei der Anwendung als 0,05 %ige Spritzbrühe eine bessere fungizide Wirkung zeigen (90 %) als die bekannten Wirkstoffe A (70 %) und B (55 %).

**Patentansprüche**

**1.** Phenylalkylamine der Formel I

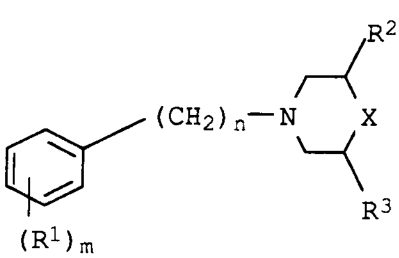

I

in der

n den Wert 5, 6, 7, 8 oder 9 hat,

m den Wert 1, 2 oder 3 hat,

$R^1$ Methyl, Halogen, Phenyl, welches bis zu drei $C_1$-$C_4$-Alkoxygruppen tragen kann, oder Phenoxy bedeutet, welches bis zu drei der folgenden Gruppen tragen kann: Halogen, $C_1$-$C_4$-Alkyl und $C_1$-$C_4$-Alkoxy,

ferner, für den Fall, daß m = 2 ist, zwei benachbarte Reste $R^1$ zusammen den Rest

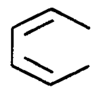

bedeuten,

X Sauerstoff bedeutet, außer wenn $R^1$ Methyl oder Chlor bedeutet, oder

X den Rest $=CR^4R^5$ bedeutet, wobei

$R^4$ Isopropyl, tert.-Butyl oder Phenyl bedeutet, welches bis zu drei der folgenden Gruppe tragen kann: Halogen, $C_1$-$C_4$-Alkyl und $C_1$-$C_4$-Alkoxy, und

$R^5$ H oder OH bedeutet,

$R^2$,$R^3$ Wasserstoff bedeuten und

$R^2$,$R^3$ für den Fall, daß X Sauerstoff bedeutet, zusätzlich Methyl oder Ethyl bedeuten

sowie deren pflanzenverträglichen Salze.

**2.** Fungizides Mittel, enthaltend einen Trägerstoff und eine fungizid wirksame Menge eines Phenylalkylamins der Formel I gemäß Anspruch 1 oder dessen pflanzenverträglichen Salze.

**3.** Verfahren zur Herstellung von Fungiziden, dadurch gekennzeichnet, daß man eine Verbindung der Formel I gemäß Anspruch 1 mit einem festen oder flüssigen Trägerstoff mischt.

**4.** Verfahren zur Bekämpfung von Pilzen, dadurch gekennzeichnet, daß man eine fungizid wirksame Menge eines Phenylalkylamins der Formel I gemäß Anspruch 1 oder dessen pflanzenverträglichen Salze auf die Pilze oder auf durch Pilzbefall bedrohte Materialien, Flächen, Pflanzen oder Saatgüter einwirken läßt.

**5.** Verbindung gemäß Anspruch 1, dadurch gekennzeichnet, daß $R^1{}_m$ 2,4-Dibrom bedeutet, n den Wert 5 hat, X Sauerstoff bedeutet und $R^2$ und $R^3$ Methyl bedeuten.

## Claims

**1.** A phenylalkylamine of the formula I

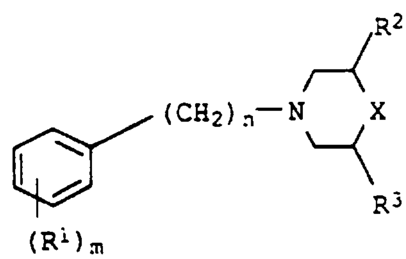

I

where

n is 5, 6, 7, 8 or 9,

m is 1, 2 or 3,

$R^1$ is methyl, halogen, phenyl which can carry up to three $C_1$-$C_4$-alkoxy groups, or phenoxy which can carry up to three of the following groups: halogen, $C_1$-$C_4$-alkyl and $C_1$-$C_4$-alkoxy,

furthermore, in the case where m is 2, two adjacent $R^1$ radicals are together

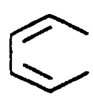

,

X is oxygen except when $R^1$ is methyl or chlorine, or
X is $=CR^4R^5$, where
$R^4$ is isopropyl, tert.-butyl or phenyl which can carry up to three of the following groups: halogen, $C_1$-$C_4$-alkyl and $C_1$-$C_4$-alkoxy, and
$R^5$ is H or OH,
$R^2$, $R^3$ are hydrogen and
$R^2$, $R^3$, in the case where X is oxygen, are additionally methyl or ethyl,
and salts thereof which are tolerated by plants.

**2.** A fungicidal composition containing a carrier and a fungicidally effective amount of a phenylalkylamine of the formula I as claimed in claim 1 or salts thereof which are tolerated by plants.

**3.** A process for preparing fungicides, which comprises mixing a compound of the formula I as claimed in claim 1 with a solid or liquid carrier.

**4.** A method for controlling fungi, which comprises a fungicidally effective amount of a phenylalkylamine of the formula I as claimed in claim 1, or salts thereof which are tolerated by plants, being allowed to act on the fungi or on materials, areas, plants or seed threatened by fungal attack.

**5.** A compound as claimed in claim 1, wherein $R^1_m$ is 2,4-dibromo, n is 5, X is oxygen and $R^2$ and $R^3$ are methyl.

**Revendications**

**1.** Phénylalkylamines de formule I

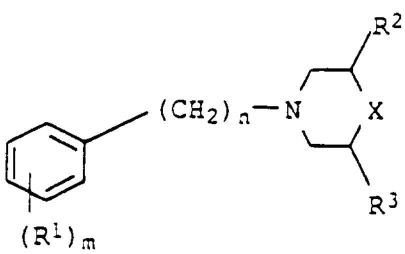

I

dans laquelle
n a la valeur 5, 6, 7, 8 ou 9,
m a la valeur 1, 2 ou 3,
$R^1$ représente méthyle, halogène, phényle qui peut porter jusqu'à trois groupes alcoxy en C1-C4 ou phénoxy qui peut porter jusqu'à trois des groupes suivants : halogène, alkyle en C1-C4 et alcoxy en C1-C4,
en outre, dans le cas où m = 2, deux restes $R^1$ voisins représentent ensemble le reste

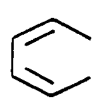

X représente oxygène, sauf quand $R^1$ signifie méthyle ou chlore, ou
X représente le reste $=CR^4R^5$,
$R^4$ représente isopropyle, tert-butyle ou phényle qui peut porter jusqu'à trois des groupes suivants : halogène, alkyle en C1-C4 et alcoxy en C1-C4, et
$R^5$ représente H ou OH,
$R^2$, $R^3$ représentent hydrogène et
$R^2$, $R^3$, dans le cas où X représente oxygène, représentent en outre méthyle ou éthyle
ainsi que leurs sels acceptables pour les plantes.

2. Agent fongicide, contenant un véhicule et une quantité efficace à effet fongicide d'une phénylalkylamine de formule I selon la revendication 1 ou de ses sels acceptables pour les plantes.

3. Procédé de préparation de fongicides, caractérisé par le fait qu'on mélange un composé de formule I selon la revendication 1 avec un véhicule solide ou fluide.

4. Procédé de lutte contre les champignons, caractérisé par le fait qu'on met en oeuvre une quantité efficace à effet fongicide d'une phénylalkylamine de formule I selon la revendication 1 ou de ses sels acceptables pour les plantes sur les champignons ou sur les matériaux, surfaces, plantes ou semences menacés d'un envahissement par les champignons.

5. Composé selon la revendication 1, caractérisé par le fait que $R^1_m$ signifie 2,4-dibrome, n vaut 5, X signifie oxygène et $R^2$ et $R^3$ méthyle.